# EUROPEAN PATENT APPLICATION

(11) **EP 4 710 842 A1**
(43) Date of publication of application: **18.03.2026**
(21) Application number: 24200927.2
(22) Date of filing: 17.09.2024
(51) Int. Cl.: A61B 5/00, G01H 9/00, G01N 29/24, H04R 23/00

(54) **A NOSE CONE, A VIBROMETRY MEASUREMENT SYSTEM AND A MANUFACTURING METHOD FOR A NOSE CONE**

(71) Applicant: Carl Zeiss Meditec AG, 07745 Jena (DE)
(72) Inventor: MacDougall, Daniel, Halifax, B3H0A8 (CA); Galbraith, Benjamin, Halifax, B3H0A8 (CA); Rahimzadeh, Arvin, Halifax, B3H 0A8 (CA); Rybakovski, Oleg, 220076 Minsk (BY)
(74) Representative: Carl Zeiss AG - Patentabteilung

(57) **Abstract**

The disclosure relates to a nose cone (160) for a vibrometry measurement system. The nose cone comprises a cone body (165) having a base portion (210) and a top portion (220); the top portion (220) comprising a plurality of ports (230, 332, 334) on a top surface (225), wherein at least one light guiding element (144) is coupled to at least one of the plurality of ports (230, 332, 334). A speaker duct (336) configured to transmit an acoustic stimulus from a speaker (152); and a microphone duct (338) configured to relay an acoustic pressure to a microphone (154). The invention further relates to a vibrometry measurement system and a method for manufacturing a nose cone.

## Description

### TECHNICAL FIELD

The present disclosure is generally directed to a configuration of a nose cone for a vibrometry measurement system. Furthermore, the disclosure is directed to a vibrometry measurement system and a manufacturing method for a nose cone.

### SUMMARY

These objects are solved by the nose cone according to claim 1, by the vibrometry measurement system according to claim 12 and by the manufacturing method of claim 14. Optional embodiments are provided in the dependent claims and the description.

In a first aspect, a nose cone for a vibrometry measurement system is provided. The nose cone includes a cone body having a base portion and a top portion. The top portion including a plurality of ports on a top surface, wherein at least one light guiding element is coupled to at least one of the plurality of ports. The speaker duct configured to transmit an acoustic stimulus from a speaker; and a microphone duct configured to relay an acoustic pressure to a microphone. The "base portion" and "top portions" are located at opposite sides of the cone body, preferably related to a symmetry or longitudinal axis of the nose cone. Particularly, the base portion has a larger diameter than the top portion of the cone body, wherein the diameter is preferably measured in a direction perpendicular to the longitudinal axis of the nose cone. Within the context of the present disclosure a "port" is defined as an interface area which connect ducts extending within the volume of the nose cone and the space external to the cone when it is operated as part of the vibrometry measurement system. Hence, ports can be considered as openings in a volume of the nose cone. The ports which are assigned to light guide elements allow light to exit the nose cone. He speaker duct directs the generated acoustic stimulus to the target structure of a patient, while the microphone duct collects the acoustic pressure from a structure. The speaker duct and the microphone duct can be built from metallic material such as steel.

In an embodiment, the speaker duct is coupled to at least another one of the plurality of ports, which might be designated as "speaker port". Alternatively, or in addition, the microphone duct is coupled to at least a different one of the plurality of ports, which might be designated as "microphone port". Within the scope of the invention there might be more than one speaker port and/or corresponding speaker duct as well as more than one microphone port and/or corresponding microphone duct. The ports, which are directed to the light elements, speaker duct and microphone duct can be shaped differently. For example, they can have a different diameter or the port of a microphone duct and/or a speaker duct can be shaped as a funnel. In this regard the diameter of the port of the speaker duct and the port of the microphone duct can be larger than the diameter of the port of the light guide element.

To provide a smooth other and inner surface of the cone body the speaker duct and/or the microphone duct is at least partly incorporated into the cone body. This will especially facilitate the mounting of the speculum tips on the cone body and will at the same time ensure an unobstructed optical path for any outgoing or incoming light.

In an embodiment, the cone body includes essentially a truncated hollow cone shape. Within the context of the present disclosure the "cone body" is defined as a volumetric body which can include cavities, recesses and/or holes. The cone body can be formed in one piece or made of several parts. The hollow shape of the nose cone results in an axial opening configured to transmit light reflected from a target structure from the top portion to the base portion. This can be used both for visible light but also for light in the non-visible rage, as is frequently used for OCT measurements. At the same time this provides a flat top surface at the top portion. This can be used to circularly arranged the plurality of ports are on a ring circle on the top surface of the top portion. Using such an arrangement ensures that all ports can be located in a common plane. Specifically, by having the speaker port and the microphone port as well as the port of the light guide element at the same plane, fewer disturbing effects happen in the acoustic system, the closest position to the inner ear structure and a higher overall resolution is achieved. Additionally, the nose cone may be rotated freely in the ear canal to a certain degree with a circular shape, which would not be possible with other configurations. However, within the scope of the present invention it is also possible that the microphone port is closer to the base portion compared to the speaker port. This might also be beneficial to reduce the risk of crosstalk between the speaker and the microscope. By minimizing the crosstalk, less sound generated by the speaker and conducted through the speaker duct and emitted into the ear canal for excitating structures within the ear to vibrate more directly reaches the microphone. That is, sound waves that are reflected from the ear back into the system reach the microphone properly. It is possible to control the intensity of the emitted sound, which further allows for minimizing crosstalk.

In an embodiment, the at least another one of the plurality of ports and the at least a different one of the plurality of ports can enclose a circular arc of more than 90°, preferably of more than 120° and more preferably of 180° from each other. In other words, the speaker port and the microphone port can be arranged more than 90°, preferably more than 120° or 180° offset to each other. Under certain circumstances this reduces the risk for crosstalking between the speaker and the microphone.

In an embodiment, the at least another one of the plurality of ports and the at least a different one of the plurality of ports are adjacent to each other. This means that between the speaker port and the microphone port no port for a light guide element is located. They speaker port and the microphone port can enclose a circular arc of less than 60°, preferably of less than 30°, more preferably of less than 20°. This facilitates the positioning of the microphone and speaker within a corresponding housing of a handheld tool.

In an embodiment, the at least one light guide element includes a plurality of optical fibers. Each of the fibers is assigned an individual port on the top portion. The optical fibers can extend beyond the base part of the nose cone and might be connected with a light source. The light source can be a single or several LEDs or a similar light source. Alternatively, the light guide element can also be a light duct provided by a hollow section within the nose cone including a reflective coating or a rigid transparent light duct.

In an embodiment, the optical fibers are evenly and/or symmetrically distributed around the perimeter of the nose cone. Preferably the ports for the optical fibers are evenly distributed by at least a circular arc of around 300°, preferably of at least around 330°. Also, this can mean that light guide elements of the same size are arranged in substantially equal distances to each other around the perimeter. Furthermore, it is also possible to limit the number of ports to e.g. two, which are exhibit an arc formed shape and which extend along a part of the perimeter, e. g. each for along 160°. Such an arc shaped port would provide an even distribution of light.

Making the cone body at least partly from an epoxy material provides an easy way to secure the light guide element, the speaker duct and the microphone duct within the cone body. As an alternative also similar means, such as glues, can be used to secure the light guide element, the speaker duct and the microphone duct within the guide body.

In yet another aspect, an improved vibrometry measurement system is provided. This system includes a nose cone according to embodiments of the present disclosure. Furthermore, it includes a speaker, which is sound transmittingly connected to a speaker duct of the nose cone, to provide an acoustic stimulus to a structure. A microphone is connected to the microphone duct of the nose cone configured to receive an acoustic pressure from a structure. At least one light guide element is configured to illuminate a target structure. In an embodiment an OCT subsystem is provided which is configured to measure a response signal from vibrations in a structure of the patient. The OCT subsystem includes an OCT light element and an OCT sensor system. In an embodiment the OCT light element from the OCT sensor system illuminates the structure through the axial opening of the nose cone. The acoustic stimulus has frequencies ranging between 125 and 8000 Hz. The target structure can be selected from a group consisting of an ossicle of a middle ear, an incus of an ear, or an umbo of the ear.

OCT is an optical interferometric imaging technology that may produce depth-resolved images of sub-surface tissue structures, such as structures within the ear. This may be accomplished by taking a spatially coherent infrared light-element and splitting it between a reference beam and a sample probing beam. Light that is backscattered from the structures within the sample is collected and interfered (combined) with the reference beam light in order to produce an interference pattern that, once processed, reveals the location of light-reflecting structures in the sample.

OCT has been applied to imaging the ear of patients. Anatomical structures within the ear may be imaged using OCT and may be used to perform functional imaging in the ear by measuring the vibration of middle ear structures in response to sound via an acoustic system. Typically, the process may include using an OCT vibrometry measurement system to extract magnitude of vibration information in non-real-time and OCT may rely on an acoustic stimulus that is applied to the ear; the acoustic frequency phase variations are then collected over many consecutive complete acoustic cycles and analyzed using Fourier analysis. The phase variations determined via the OCT vibrometry measurement system may then be displayed to a user, such as a medical professional, doctor, or nurse.

Such display options of phase variations may include a display device, such as a computer. The display may illustrate measurements, images collected, information regarding the measurements, and provide feedback to the user regarding the measurements. By utilizing the display, all information may be available to the user at the same time in the same location. The user may then be able to determine more on the measurements, based on direct feedback via a display. For example, once measurements are displayed to the user, the user may be able to determine issues or diseases of the ear based on the measurements.

In yet another aspect a method for manufacturing a nose cone for a vibrometry measurement system is provided. The method includes the steps of providing a cone shaped mounting part. The mounting part has a plurality of recesses on its outer surface configured for mounting a light guide element, a speaker tube and a microphone tube. During the manufacturing the light guide element, the speaker tube and the microphone tube are inserted into the recesses of the mounting part. The light guide element, the speaker tube and the microphone tube are secured in the recesses by means of a moulding step. The recesses can be formed to have a diameter which is large enough to incorporate the light guide element, the speaker tube and the microphone tube. The speaker tube and the microphone tube can be formed as metallic tubes which form the speaker duct, configured to provide acoustic stimulus from a speaker, and the microphone duct configured to relay an acoustic pressure to a microphone. To avoid entering of material during the moulding step into the tubes, wires can be inserted into the tubes before applying the moulding material such as epoxy. Within the meaning of the present disclosure the molding step includes applying moldable martials such as epoxy or plastic, preferably by means of a mould. Having recesses, which are open to the outside of the mounting part, facilitates the mounting of the tubes and light guide element, as they can simply inserted radially into the recesses or grooves. If the light guide element consists of a plurality of optical fibers each of the fibers will be accommodated in one of the recesses. It is possible that recesses with different diameters are provided as the diameter of the light guide element, which might be optical fibers, might be different than the diameter of the speaker tube and the microphone tube.

The positioning of the light guide element, the speaker tube and the microphone tube is facilitated if an auxiliary mounting portion is providing. This auxiliary mounting portion provides radial support of the light guide element, the speaker tube, and/or the microphone tube. The light guide element, the speaker tube and/or the microphone tube can be axially fixed at the auxiliary mounting portion. This can be achieved by gluing the light guide elements and the tubes into the auxiliary mounting portion by using a suitable glue. The auxiliary mounting portion can be an integral part of the mounting part separate part which might be connected with the mounting part prior to the moulding and separated from it after the curing of the moulded material.

### BRIEF DESCRIPTION OF THE FIGURES

The accompanying drawings are incorporated herein and form a part of the specification.
FIG. 1 illustrates a system of a configuration of a nose cone with a vibrometry measurement system, according to some embodiments.
FIG. 2 illustrates a side view of a configuration of a nose cone for insertion in an ear, according to some embodiments.
FIG. 3 illustrates a front view of a configuration of a nose cone for insertion in an ear, according to some embodiments.
FIG. 4A illustrates a perspective view of a configuration of a nose cone for insertion in an ear, according to some embodiments.
FIG. 4B illustrates a side view of a configuration of a nose cone for insertion in an ear, according to some embodiments.
FIG. 5A illustrates a perspective view of a configuration of a nose cone for insertion in an ear, according to some embodiments.
FIG. 5B illustrates a perspective view of a configuration of a nose cone for insertion in an ear, according to some embodiments.
FIG. 6 illustrates a perspective view of a configuration of a nose cone including cabling for insertion in an ear, according to some embodiments.
FIG. 7 illustrates a method for a configuration of a nose cone for a vibrometry measurement system, according to some embodiments.
FIG. 8 illustrates a perspective view of a mounting part for manufacturing a nose cone, according to some embodiments.
FIG. 9 illustrates a method of a manufacturing process of a nose cone, according to some embodiments.
FIG. 10 illustrates an example computer system useful for implementing various embodiments, according to some embodiments.

In the drawings, like reference numbers generally indicate identical or similar elements. Additionally, generally, the left-most digit(s) of a reference number identifies the drawing in which the reference number first appears.

### DETAILED DESCRIPTION

Otoscopy is a well-known clinical procedure used to examine structures within the ear canal of a patient, including the ear drum. A nose cone of an otoscope is inserted into a patient's ear to assess the ear. However, otoscopy has its limitations, such as poor visualization or inadequate time for evaluation in suboptimal environments. Especially its limitation to the ear canal is disadvantageous as the middle ear cannot be assessed using conventional otoscopy. Recently, new devices have been developed to improve early and reliable diagnosis of various ear diseases and facilitate the view behind the ear drum to allow for spatially resolved and three-dimensional measurements of structures within the middle ear. Newer technologies may be used for performing diagnostic procedures using a combination of vibrometry techniques and imaging of the ear. In this regard a promising approach has been the combination of vibrometry and optical coherence tomography (OCT) imaging. However also other imaging methods such as spectroscopy measurements can be used.

For classical otoscopy devices it is necessary to provide illumination to the ear and at the same time it is required to allow visible inspection of the target area, i.e. the ear canal and/or ear drum by the medical personnel, i.e. the doctor assessing the ear of a patient. This visible inspection can be facilitated either by means of a magnifying optics or by a camera, capturing the light reflected from the target area which is then displayed onto a screen. This allows for a rather simple design of the nose cone, and it must only be ensured that the components guiding the light to the target do not obstruct the optical path of the reflected light to the eye of the medical personnel or the camera.

However, for systems combining OCT and vibrometry as well a classical imaging - similar to classical otoscopy - nose cones from classical otoscopy cannot be used, as it is necessary to guide not only light but also sound to the ear of the patient and also to detect reflected sound via a microphone. At the same time, it is crucial that the optical path of the OCT light source is not obstructed.

Therefore, the objective technical problem to provide an improved nose cone for a vibrometry measurement system, an improved vibrometry measurement system and an improved method for manufacturing an improved nose cone.

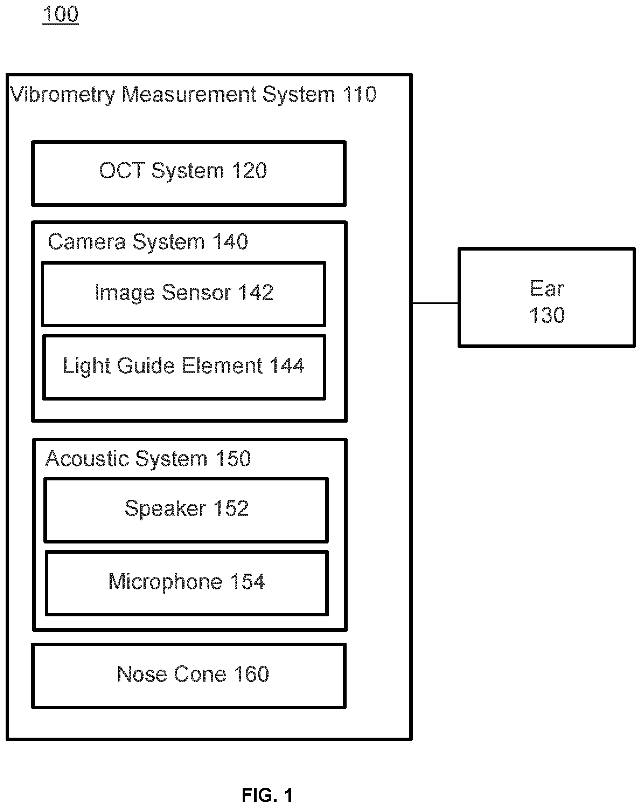

FIG. 1 illustrates a system 100 for a vibrometry measurement system 110, according to some embodiments. System 100 includes a vibrometry measurement system 110 including an OCT system 120, a camera system 140, an acoustic system 150, and a nose cone 160 to be used to measure structures within an ear 130.

In some embodiments, the vibrometry measurement system 110 may be used by a user, such as a medical professional, a doctor, or a nurse, may be handheld, and be inserted into the ear 130, via the nose cone 160, of a patient to perform various measurements on tissues within the ear 130. The vibrometry measurement system 110 may include, but is not limited to, an OCT system 120, a camera system 140, an acoustic system 150, and a nose cone 160. The acoustic system 150 may include a speaker 152 and a microphone 154.

The acoustic system 150 may generate an acoustic stimulus, via the speaker 152, at a plurality of frequencies within the ear 130. The frequencies of the acoustic stimulus may range between 125 and 8000 Hz. The acoustic system 150 may be used to measure the sound pressure levels, via the microphone 154, of the frequencies near structures within the ear 130. While system 100 is described with reference to one speaker 152 and one microphone 154, more speakers and microphones may be envisioned. For example, two speakers may be utilized with one microphone to generate frequencies at different locations in the ear 130. Another example may include two microphones and one speaker such that more sound pressure levels may be measured.

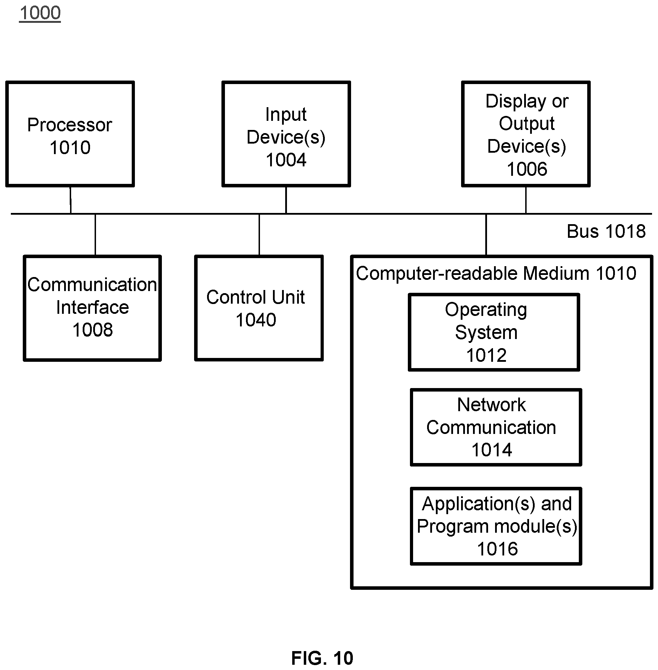

The OCT system 120 may include an OCT light element and an OCT sensor system, which includes an interferometer configured to generate a sample beam and reference beam, and a detector to detect an interfered reference beam and scattered light, a scanning system for scanning the sample beam on the region of interest, such as the ear 130. Based on the acoustic stimulus generated by the acoustic system 150, the OCT system 120 may receive signals and generate images and may be displayed to the user via a display 1006, further described in FIG. 10.

When using the OCT system 120 to generate images, the images may correspond to a B-mode image of the ear 130 and may include lines to indicate the depth selected for imaging. The B-mode image may be altered by the acoustic stimulus generated from the acoustic system 150 and is based on a plurality of A-scans from the OCT system 120, and a plurality of sample interferograms.

Based on the frequency used during a measurement of the ear 130, a mobility measurement of the inner ear may be determined. The mobility measurement may be based on an amplitude of the vibration induced on the structure from the acoustic system 150 and sound pressure driving the vibration of the structure in the ear 130 at the frequency. The structure in the ear 130 may be the malleus, incus, stapes, or umbo. The mobility measurement may be measured multiple times at multiple frequencies thereby generating a plurality of mobility measurements and displayed to the user on the display 1006. The mobility measurement can be described in terms of acceleration of those structures in the ear 130 with regards to frequency response, the magnitude of structure displacement normalized to ear canal pressure versus frequency.

In some embodiments, a camera system 140 may be used simultaneously with the OCT system 120. The camera system 140 may include an image sensor 142 and a light guide element 144. The image sensor 142 may be a charged coupled device (CCD), video, thermal, or infrared camera and may capture and record real-time images of structures or tissues within the ear 130. The camera system 140 may share a common beam path with the OCT system 120 such that the same region of interest is imaged within the ear 130.

The camera system 140 may generate images and may be displayed in the display. The images may display the tympanic membrane, malleus, incus, stapes, or umbo of the ear 130 of the patient. While the embodiments described herein are related to the ear, measuring across other tissues in the human body may be envisioned and realized using techniques described herein.

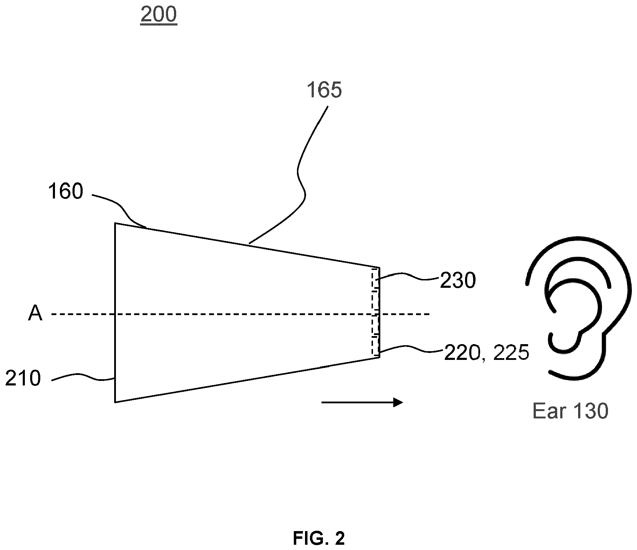

The nose cone 160 has a conical configuration and is further illustrated in FIGS. 2-6. The nose cone 160 as described herein may improve the user's ability to measure within the ear 130 and detect diseases. Previous nose cones are not equipped with both lighting and sound generating, transmitting, and relaying capabilities as the nose cone 160 described herein. Reflections on structures via the nose cone 160 within the ear 130 from both light and sound allow for assessment and diagnoses of diseases within the ear 130. With the speaker 152, microphone 154, and light guide element 144 converging on the nose cone 160, the beam path in the opening of the nose cone 160 is free and has not disturbances. The outlet and inlet openings of speaker 152, microphone 154, and light guide element 144 may be at the same plane but may also be at different locations on the nose cone 160.

Additionally, commercially available speculum tips may be disposed over the nose cone 160 for actual measuring within the ear 130. These speculum tips may be reusable or disposable and have known sizes based on the age of the patient. For example, typical sizes of a speculum tip may include 2.4 mm for a baby or a child, 3 mm for a child or teenager, 4 mm for a teenager or an adult, and 5 mm for an adult or large adult. These commercially available speculum tips may fit directly over nose cone 160. By utilizing the nose cone 160 described herein with a commercially available speculum tip, cross-contamination may be reduced, while still being able to see directly into the ear 130 and detect diseases or problems within the ear 130.

By utilizing such a nose cone 160 in conjunction with the OCT system 120, camera system 140, and acoustic system 150 as described herein, clear visibility of the ear 130 during measurements is possible. The nose cone 160 may function seamlessly with commercially available speculum tips and may provide excellent illumination and magnification for a detailed view of structures within the ear 130 that a traditional otoscope can not provide.

The illumination from either the OCT light element or the light guide element 144 may be independent and have separate channels from the both the speaker 152 and the microphone 154. In addition to separate channels, a solid material (not pictured) may separate the speaker 152 and microphone 154. However, outlet and inlet openings of the light guide element 144, speaker 152, and microphone 154 may be in the same plane of the nose cone 160, as seen in FIGS. 2-6, but may also be in different planes.

FIG. 2 illustrates a configuration 200 of a nose cone 160 for insertion in an ear 130, according to some embodiments. The nose cone 160 may include a base portion 210, a top portion 220, and a plurality of ports 230.

In some embodiments, the nose cone 160 may have a cylindrical cone configuration and includes a base 210 and a top 220. The nose cone 160 may be hollow, made from epoxy, and may be translucent. The nose cone 160 may have a plurality of ports 230 that may receive any one of a light guide element 144, a speaker 152, or a microphone 154. While the plurality of ports 230 appear indented within the nose cone 160, that is merely for illustration purposes and the plurality of ports 230 are on the top 220 of the nose cone 160 and are flush with the top 220.

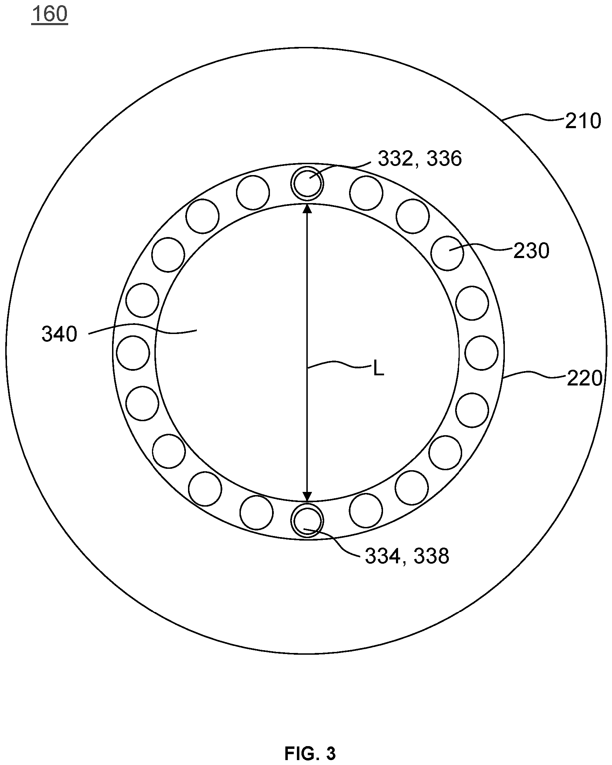

FIG. 3 illustrates a configuration of a top view of a nose cone 160 for insertion in an ear 130, according to some embodiments. The nose cone 160 may include a base 210, a top 220, a plurality of ports 230, port 332, and port 334.

The plurality of ports 230 may receive a light guide element 144 and the light guide element 144 may be embedded in the nose cone 160. For example, the light guide element 144 may be embedded in at least one of the ports 230 at the top 220 of the nose cone 160. The light guide element 144 may be, for example, an optical fiber, light guide, LED, laser, or the like.

The speaker 152 may be used to deliver sounds via port 332 or port 334, depending on the configuration, to vibrate the ear 130. The frequencies of the sound may be between 125 and 8000 Hz, which is considered the typical hearing range for hearing tests of the ear 130. The microphone 154 may be used to measure the sound pressure levels near structures within the ear 130. The light guide element 144 may be used to illuminate the inside of the ear 130. A speculum tip may fit over the nose cone 160 and then may be directly inserted into the ear 130.

In some embodiments, the sound exit (i.e. output duct) for the speaker 152 and the sound entry point (i.e. input duct) for the microphone 154 may be embedded in the nose cone 160. For example, wiring for both the speaker 152 and the microphone 154 may be embedded at either port 332 or port 334 at the top 220 of the nose cone 160. By having the microphone 154 and speaker 152 separated by a length L, crosstalk between the two may be minimized and to that effect, prevented. Other embodiments may be envisioned and are further described herein.

For example, the length of the nose cone 160 may be less than 30 mm, preferably less than 25 mm. The width of the top 220 may be less than 10 mm, preferably less than 6 mm. The length L may be less than 5 mm, preferably less than 3.5 mm.

In some embodiments, by having the sound exit point of the speaker 152 and the sound entry point of the microphone 154 in the same plane, but offset by 180°, crosstalk may be minimized. The entry point and exit point of sound are at the maximum distances from each other, such that the distance L is maximized.

By having other configurations of the speaker 152 or microphone 154, a disturbance, such as crosstalk, may cause the electric or magnetic fields of the signal of either one of the microphone 154 or the speaker 152 to affect the signal of the other. The signal from the microphone 154 may leak into the signal from the speaker 152, or vice versa. For example, if the speaker 152 and the microphone 154 are directly adjacent to each other or only separated by a few ports 230, the possibility of crosstalk is increased. The sound signal of the speaker 152 may leak into the sound signal of the microphone 154 or vice versa, which may skew the results of the measurements of the ear 130. The signals may blend together, which may affect the measurement and lead to false measurements within the ear 130 of the patient. Additionally, if the microphone 154 is near the field of the speaker 152, the pressure measurement for the mobility measurement will not be correct. Hence, it is advantageous to have a maximum distance between the speaker 152 and the microphone 154.

While the distance between the speaker 152 and the microphone 154 should be maximized, the other ports 230 may be embedded with a light guide element 144. The light guide element 144 may provide light into the vibrometry measurement system 110 such that imaging may be performed by the camera system 140. While a plurality of ports 230 are illustrated, more or less may be envisioned depending on the desired light output from the light guide element 144.

The light guide element 144 may be a plurality of optical fibers. Optical fibers may be advantageous due to the lack of crosstalk between the signals of the cables of the optical fibers, nor does it heat up. Additionally, the optical fiber does not pick up background noise. Additionally, other types of light guide element 144 may be envisioned other than light fibers.

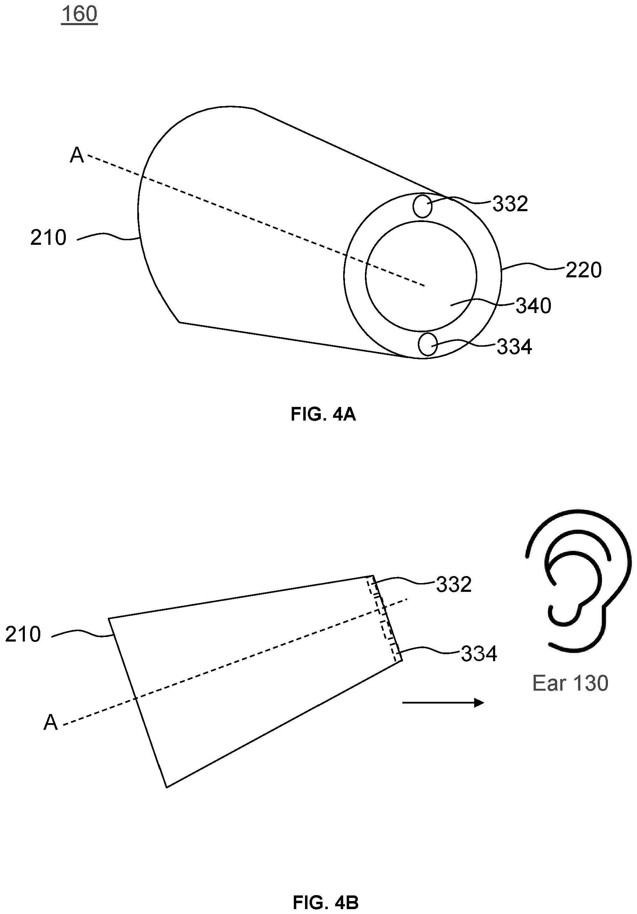

FIG. 4A illustrates a perspective view of a configuration of a nose cone 160 for insertion in an ear 130, according to some embodiments. The nose cone 160 includes a base 210 and a top 220 with port 332, port 334, and opening 340.

As described herein, either the speaker 152 or the microphone 154 may be in either port 332 or port 334. In some embodiments, the microphone 154 should be at port 332 and the speaker 152 should be at port 334 because the acoustic stimulus should be closer to a specific structure in the ear 130 such as the malleus, incus, stapes, or umbo. By having the exit point of the sound from the speaker 152 being closer to the ear 130, the sound may have a focused output. While not explicitly illustrated in FIG. 4A, the rest of the top 220 may include port 230 for light guide element 144. The light guide element 144 may be distributed along the top 210 such that light is output homogenously.

At opening 340, light from the OCT light element may exit the nose cone 160 to illuminate the structure in the ear 130 and be reflected back through the opening 340, eventually hitting the detector of the OCT system 120. Additionally, light from the light guide element 144 may illuminate the structure in the ear 130 and be reflected back through the opening 340, eventually hitting the image sensor 142.

FIG. 4B illustrates a side view of a configuration of a nose cone 160 for insertion in an ear, according to some embodiments. The nose cone 160 includes a base portion 210 and a top portion 220 with port 332 and port 334.

Due to the vibrometry measurement system 110 being in the hand of the user, the insertion of the vibrometry measurement system 110 into the ear 130 may not be at the most desirable angle. Due to the position of the vibrometry measurement system 110 not entering the ear 130 at the correct angle, the position of the speaker 152 is more critical to maximize the output of the acoustic stimulus.

In some embodiments, the microphone 154 should be at port 332 and the speaker 152 should be at port 334 because the acoustic stimulus should be closer to a specific structure in the ear 130, such as the malleus, incus, stapes, or umbo. By having the exit point of the sound from the speaker 152 being closer, the sound may have a focused output.

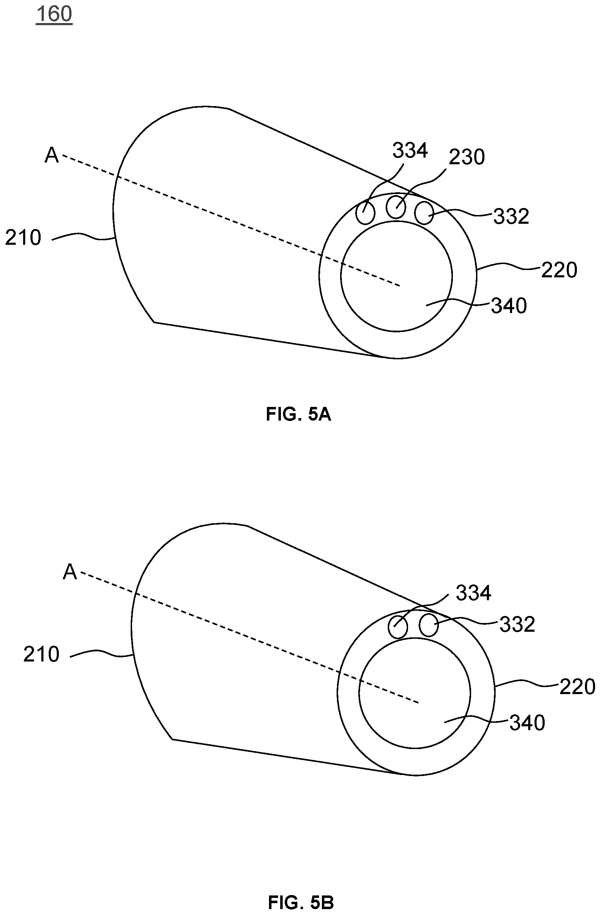

FIG. 5A illustrates a perspective view of a configuration of a nose cone for insertion in an ear, according to some embodiments. The nose cone 160 includes a base portion 210 and a top portion 220 with port 230, port 332, and port 334.

In some embodiments, the port 230 for light guide element 144 may be between the port 332 and port 334, which may include either the speaker 152 or microphone 154. While not illustrated in FIG. 5A, the rest of the top 220 may include a plurality of ports 230 for light guide element 144. The light guide element 144 may be distributed along the top 210 such that light is output homogenously. For example, by having the speaker 152 and microphone 154 side-by-side, a more compact design may be achieved, resulting also in easier manufacturing of the nose cone 160.

FIG. 5B illustrates a perspective view of a configuration of a nose cone for insertion in an ear, according to some embodiments. The nose cone 160 includes a base 210 and a top 220 with port 332 and port 334.

In some embodiments, the port 230 for light guide element 144 may be surrounding the port 332 and port 334, which may include either the speaker 152 or microphone 154. While not illustrated in FIG. 5A, the rest of the top 220 may include a plurality of ports 230 for light guide element 144. The light guide element 144 may be distributed along the top 220 such that light is output in homogenously.

The superimposition of the individual illumination regions from a plurality of ports 230 with light guide element 144 may lead to an inhomogeneous total illumination, with the central region being more strongly illuminated than the outer regions. A shadow may be cast by the lens of the OCT system 120, which may be inserted into the opening 340, which may lead to an overall more homogeneous illumination.

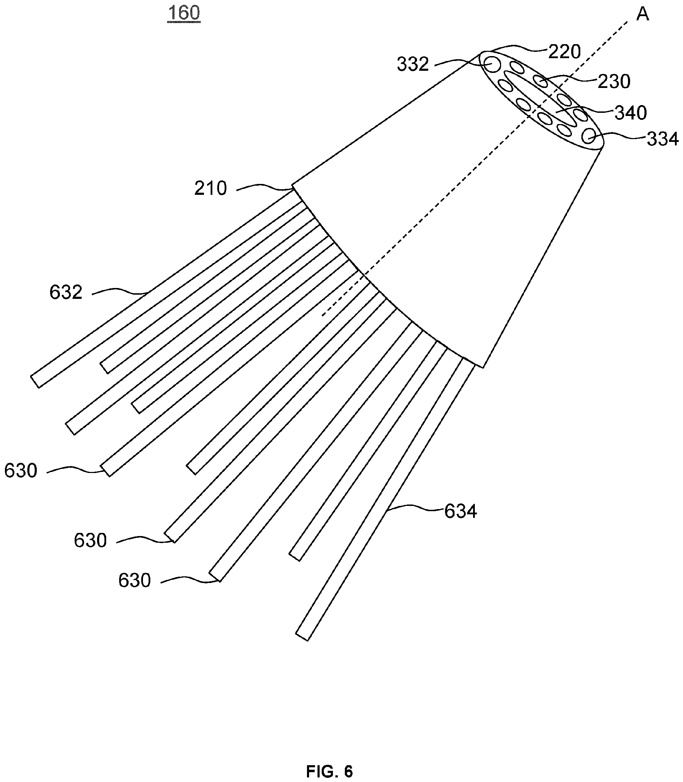

FIG. 6 illustrates a perspective view of a configuration of a nose cone 160 including cabling for insertion in an ear 130, according to some embodiments. The nose cone 160 may include a base 210, a top 220 including ports 230, port 332, port 334, and opening 340, and microphone cabling 632, speaker cabling 634, and cabling 630.

As described herein, either the speaker 152 or the microphone 154 may be embedded in either port 332 or port 334. The light guide element 144 may be embedded in at least one of the plurality of ports 230. The nose cone 160 may be cylindrical and hollow inside. By having the nose cone 160 as a cylindrical tube, it allows for easy insertion into the ear canal of the ear 130 and provides a magnified view of structures within the ear 130. The cross-section view of the nose cone 160 illustrates that the inside is hollow. The port 230 is at the top 220 of the nose cone 160.

Cabling 630 may be for the light guide element 144, which may be a plurality of optical fibers. The cabling 630 may be connected to circuitry (not pictured) to provide signals between a computer system 1000, further described in FIG. 10. The microphone cabling 632 may provide signals from the computer system 1000 and the microphone 154. The speaker cabling 634 may provide signals from the computer system 1000 and the speaker 152.

For illustrative purposes, the microphone cabling 632 is shown to be for the microphone 154 and the speaker cabling 634 is shown to be for the speaker 152. However, the microphone cabling 632 may also be for the speaker 152 and the speaker cabling 634 may be for the microphone 154. Additionally, various configurations for the cabling may change based on the embodiments described herein and illustrated in FIG. 5.

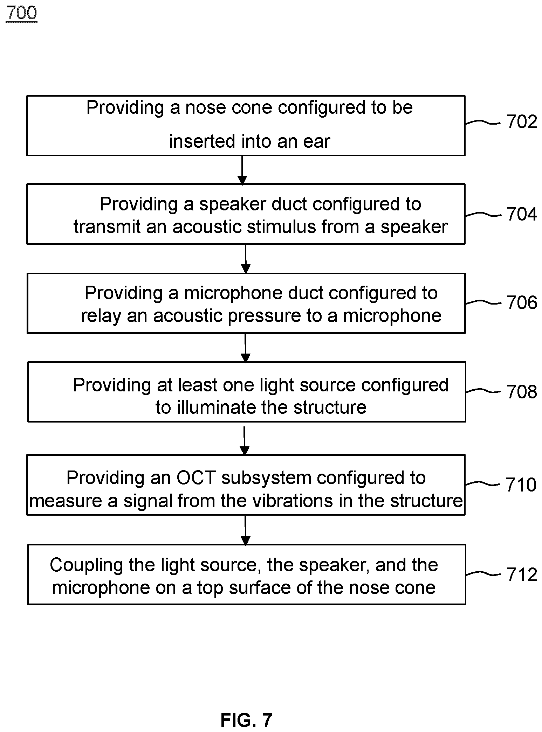

FIG. 7 illustrates a method 700 for a configuration of a nose cone for a vibrometry measurement system, according to some embodiments. Method 700 may be performed by processing logic that may include hardware (e.g., circuitry, dedicated logic, programmable logic, microcode, etc.), software (e.g., instructions executing on a processing device), or a combination thereof. It is to be appreciated that not all steps may be needed to perform the disclosure provided herein. Further, some of the steps may be performed simultaneously, or in a different order than shown in FIG. 7, as will be understood by a person of ordinary skill in the art.

Method 700 shall be described with reference to FIGS. 1-6. However, method 700 is not limited to that example embodiment.

In step 702, a nose cone configured to measure a structure may be provided. The nose cone may be coated with an epoxy material. For example, nose cone 160 may be provided. The nose cone 160 may be provided in a system and may measure a structure, such as the malleus, incus, stapes, or umbo within the ear 130. The nose cone 160 may be coated with an epoxy material such as a polymer, epoxy resin, thermoplastic, or the like.

In step 704, a speaker configured to generate an acoustic stimulus may be provided. For example, the speaker 152 may be provided and configured to generate an acoustic stimulus. The acoustic stimulus may have frequencies ranging between 125 and 8000 Hz.

In step 706, a microphone configured to measure the acoustic stimulus may be provided. For example, a microphone 154 may be provided and configured to measure the acoustic stimulus.

In step 708, the speaker and the microphone may be embedded in the nose cone. The embedding may further include gluing the speaker and the microphone at the top of the nose cone.

For example, the speaker 152 and the microphone 154 may be embedded in the nose cone 160. The speaker 152 and the microphone 154 may be glued at the top 220 of the nose cone 160.

In step 710, at least one light guide element 144 configured to illuminate the structure may be provided. The method may further include embedding the plurality of optical fibers on the top of the nose cone such that the form a circle on the top. The at least one light guide element 144 may illuminate the structure through an opening of the nose cone. For example, a light guide element 144 may be provided and may illuminate the structure within the ear 130. The light guide element 144 may be a plurality of optical fibers. The plurality of optical fibers may be embedded on the top 220 of the nose cone 160 such that the form a circle on the top 220. The at least one light guide element 144 may illuminate the structure on the ear 130 through an opening 340 of the nose cone160.

In step 712, an OCT subsystem configured to measure a signal from vibrations in the structure may be provided. The method may further include illuminating the structure through an opening of the nose cone via the OCT light element. For example, an OCT system 120 may be provided and may be configured to measure a signal from vibrations in the structure of the ear 130. The structure in the ear 130 may be further illuminated through an opening 340 of the nose cone 160 via the OCT light element.

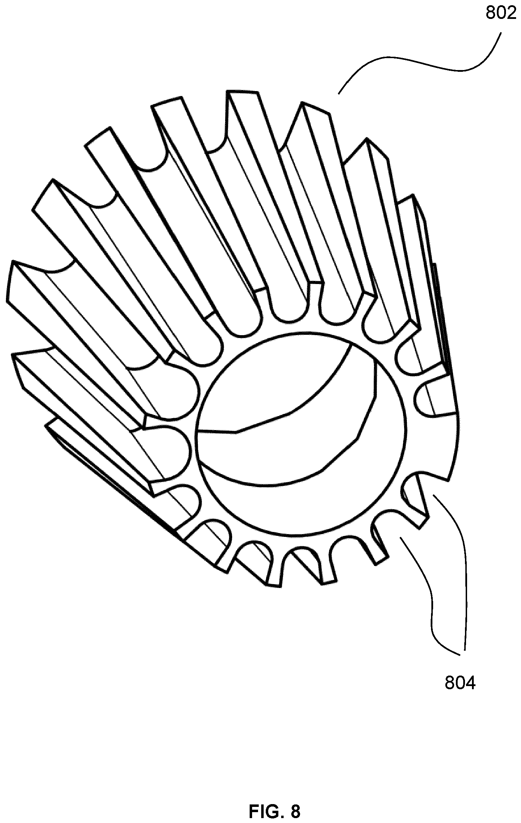

FIG. 8 shows a perspective view of a mounting part 802 for manufacturing a nose cone, according to some embodiments. The mounting part 802 includes a coned shape and includes a plurality of recesses 804 on its outer surface. The recesses 804 can accommodate the optical fibers of a light guide element 144, a speaker tube and a microphone tube. After the light guide element 144, the speaker tube and the microphone tube are inserted into the recesses 904 of the mounting part 802, the light guide can be fixed at a top portion of an auxiliary mounting portion, which facilitates the alignment of the optical fibers.

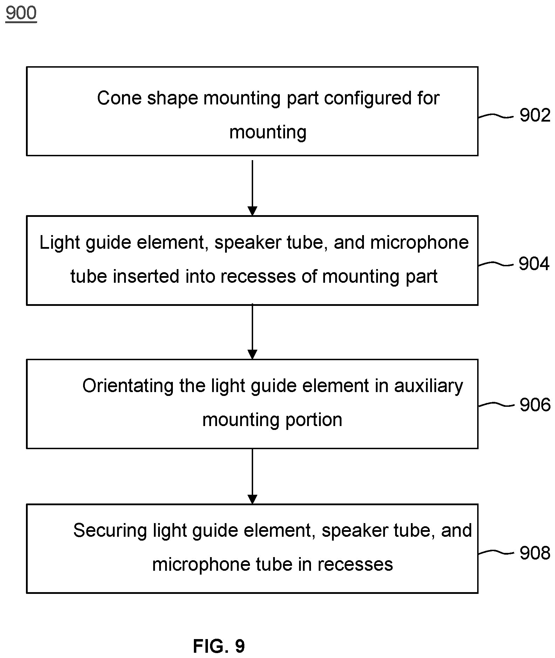

FIG. 9 illustrates a method 900 of a manufacturing method for a nose cone 160, according to some embodiments. In a step 902, a cone shaped mounting part 802 having a plurality of recesses 804 on its outer surface configured for mounting a light guide element 144, a speaker tube and a microphone tube is provided. In a step 904, the light guide element 144, the speaker tube and the microphone tube are inserted into the recesses 804 of the mounting part 802. In step 906, the light guide element 144 is oriented in an auxiliary mounting portion providing radial support. In step 908, the light guide element 144, the speaker tube and the microphone tube are secured in the recesses by means of a molding step. After the curing, the mold can be removed. During the molding additional parts of the nose cone 160 can be molded together.

Various embodiments may be implemented, for example, using one or more well-known computer systems, such as computer system 1000 shown in FIG. 10. For example, the vibrometry measurement system 110 may be implemented using combinations or sub-combinations of computer system 1000. Also, or alternatively, one or more computer systems 1000 may be used, for example, to implement any of the embodiments discussed herein, as well as combinations and sub-combinations thereof.

FIG. 10 shows a computer device 1000 for implementing various embodiments, according to some embodiments. For example, computer device 1000 may function as system of the vibrometry measurement system 110 or any portion(s) thereof, or multiple computer devices 1000 may function as a system of the vibrometry measurement system 110.

Computer device 1000 may be implemented on any electronic device that runs software applications derived from compiled instructions, including without limitation personal computers, servers, smart phones, media players, electronic tablets, game consoles, email devices, etc. In some implementations, computer device 1000 may include one or more processors 1010, one or more input devices 1004, one or more display devices 1006, one or more communication interfaces 1008, and one or more computer-readable medium 1010. Each of these components may be coupled by bus 1018, and in some embodiments, these components may be distributed among multiple physical locations and coupled by a network.

Control unit 1040 may assist in providing instructions to the vibrometry measurement system 110. The control unit 1040 may send an electronic control signal to the vibrometry measurement system 110. In response to receiving the control signal, the vibrometry measurement system 110 may perform measurements. The surgeon or another user may design a configuration of the control unit 1040 by creating the instructions and providing the instructions to the computer device 1000. For example, the instructions may be uploaded to a database of the computer device 1000 or the surgeon may use input device 1004 to perform measurements using the vibrometry measurement system 110.

Display device 1006 may be any known display technology, including but not limited to display devices using Liquid Crystal Display (LCD) or Light Emitting Diode (LED) technology. Processor(s) 1010 may use any known processor technology, including but not limited to graphics processors and multi-core processors. Input device 1004 may be any known input device technology, including but not limited to a keyboard (including a virtual keyboard), mouse, controller, joystick, track ball, and touch-sensitive pad or display.

Bus 1018 may be any known internal or external bus technology, including but not limited to ISA, EISA, PCI, PCI Express, NuBus, USB, Serial ATA or FireWire. In some embodiments, some or all devices shown as coupled by bus 1018 may not be coupled to one another by a physical bus, but by a network connection, for example. Computer-readable medium 1010 may be any medium that participates in providing instructions to processor(s) 1010 for execution, including without limitation, non-volatile storage media (e.g., optical disks, magnetic disks, flash drives, etc.), or volatile media (e.g., SDRAM, ROM, etc.).

Computer-readable medium may include various instructions for implementing an operating system 1012 (e.g., Mac OS, Windows, Linux). The operating system 1012 may be multi-user, multiprocessing, multitasking, multithreading, real-time, and the like. The operating system 1012 may perform basic tasks, including but not limited to: recognizing input from input device 1004; sending output to display device 1006; keeping track of files and directories on computer-readable medium 1010; controlling peripheral devices (e.g., disk drives, printers, etc.) which may be controlled directly or through an I/O controller; and managing traffic on bus 1018. Network communication 1014 may establish and maintain network connections (e.g., software for implementing communication protocols, such as TCP/IP, HTTP, Ethernet, telephony, etc.).

Application(s) and program module(s) 1016 may be an application that uses or implements the outcome of processes described herein and/or other processes. For example, application(s) 1016 may provide UI and/or UI elements for displaying and/or manipulating updates identified by computer device 1000 as described above. In some embodiments, the various processes may also be implemented in operating system 1012.

The described features may be implemented in one or more computer programs that may be executable on a programmable system including at least one programmable processor coupled to receive data and instructions from, and to transmit data and instructions to, a data storage system, at least one input device, and at least one output device. A computer program is a set of instructions that may be used, directly or indirectly, in a computer to perform a certain activity or bring about a certain result. A computer program may be written in any form of programming language (e.g., Objective-C, Java), including compiled or interpreted languages, and it may be deployed in any form, including as a stand-alone program or as a module, component, subroutine, or other unit suitable for use in a computing environment.

Suitable processors 1010 for the execution of a program of instructions may include, by way of example, both general and special purpose microprocessors, and the sole processor or one of multiple processors or cores, of any kind of computer. Generally, a processor 1010 may receive instructions and data from a read-only memory or a random-access memory or both. The essential elements of a computer may include a processor 1010 for executing instructions and one or more memories for storing instructions and data. Generally, a computer may also include, or be operatively coupled to communicate with, one or more mass storage devices for storing data files; such devices include magnetic disks, such as internal hard disks and removable disks; magneto-optical disks; and optical disks. Storage devices suitable for tangibly embodying computer program instructions and data may include all forms of non-volatile memory, including by way of example semiconductor memory devices, such as EPROM, EEPROM, and flash memory devices; magnetic disks such as internal hard disks and removable disks; magneto-optical disks; and CD-ROM and DVD-ROM disks. The processor and the memory may be supplemented by, or incorporated in, ASICs (application-specific integrated circuits).

To provide for interaction with a user, the features may be implemented on a computer having a display device such as an LED or LCD monitor for displaying information to the user and a keyboard and a pointing device such as a mouse or a trackball by which the user may provide input to the computer.

The features may be implemented in a computer system that includes a back-end component, such as a data server, or that includes a middleware component, such as an application server or an Internet server, or that includes a front-end component, such as a client computer having a graphical user interface or an Internet browser, or any combination thereof. The components of the system may be connected by any form or medium of digital data communication such as a communication network. Examples of communication network include, e.g., a telephone network, a LAN, a WAN, and the computers and networks forming the Internet.

The computer system may include clients and servers. A client and server may generally be remote from each other and may typically interact through a network. The relationship of client and server may arise by virtue of computer programs running on the respective computers and having a client-server relationship to each other.

One or more features or steps of the disclosed embodiments may be implemented using an API and/or SDK, in addition to those functions specifically described above as being implemented using an API and/or SDK. An API may define one or more parameters that are passed between a calling application and other software code (e.g., an operating system, library routine, function) that provides a service, which provides data, or that performs an operation or a computation. SDKs may include APIs (or multiple APIs), integrated development environments (IDEs), documentation, libraries, code samples, and other utilities.

The API and/or SDK may be implemented as one or more calls in program code that send or receive one or more parameters through a parameter list or other structure based on a call convention defined in an API and/or SDK specification document. A parameter may be a constant, a key, a data structure, an object, an object class, a variable, a data type, a pointer, an array, a list, or another call. API and/or SDK calls and parameters may be implemented in any programming language. The programming language may define the vocabulary and calling convention that a programmer will employ to access functions supporting the API and/or SDK.

In some implementations, an API and/or SDK call may report to an application the capabilities of a device running the application, such as input capability, output capability, processing capability, power capability, communications capability, etc.

While this disclosure describes exemplary embodiments for exemplary fields and applications, it should be understood that the disclosure is not limited thereto. Other embodiments and modifications thereto are possible and are within the scope and spirit of this disclosure. For example, and without limiting the generality of this paragraph, embodiments are not limited to the software, hardware, firmware, and/or entities illustrated in the figures and/or described herein. Further, embodiments (whether or not explicitly described herein) have significant utility to fields and applications beyond the examples described herein.

Embodiments have been described herein with the aid of functional building blocks illustrating the implementation of specified functions and relationships thereof. The boundaries of these functional building blocks have been arbitrarily defined herein for the convenience of the description. Alternate boundaries can be defined as long as the specified functions and relationships (or equivalents thereof) are appropriately performed. Also, alternative embodiments can perform functional blocks, steps, operations, methods, etc. using orderings different than those described herein.

References herein to "one embodiment," "an embodiment," "an example embodiment," or similar phrases, indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of persons skilled in the relevant art(s) to incorporate such feature, structure, or characteristic into other embodiments whether or not explicitly mentioned or described herein. Additionally, some embodiments can be described using the expression "coupled" and "connected" along with their derivatives. These terms are not necessarily intended as synonyms for each other. For example, some embodiments can be described using the terms "connected" and/or "coupled" to indicate that two or more elements are in direct physical or electrical contact with each other. The term "coupled," however, can also mean that two or more elements are not in direct contact with each other, but yet still co-operate or interact with each other.

The breadth and scope of this disclosure should not be limited by any of the above-described exemplary embodiments, but should be defined only in accordance with the following claims and their equivalents.

### List of reference signs

- 100: system
- 110: vibrometry measurement system
- 120: OCT System
- 130: ear
- 140: camera system
- 142: image sensor
- 144: light guide element
- 150: acoustic system
- 152: speaker
- 154: microphone
- 160: nose cone
- 165: cone body
- 200: configuration of nose cone
- 210: base portion
- 220: top portion
- 225: top surface
- 230, 332, 334: port
- 336: speaker duct
- 338: microphone duct
- 340: opening
- 702-712: method steps
- 802: mounting part
- 804: recess
- 900: method for manufacturing a nose cone
- 902-908: process steps
- 1000: computer device
- 1004: input device
- 1006: display
- 1010: processor
- 1012: operating system
- 1014: network communication
- 1016: application and program module
- 1040: control unit

## Claims

1. A nose cone (160) for a vibrometry measurement system comprising:
a cone body (165) having a base portion (210) and a top portion (220);
the top portion (220) comprising a plurality of ports (230, 332, 334) on a top surface (225), wherein at least one light guiding element (144) is coupled to at least one of the plurality of ports (230, 332, 334);
a speaker duct (336) configured to transmit an acoustic stimulus from a speaker (152); and
a microphone duct (338) configured to relay an acoustic pressure to a microphone (154).

2. The nose cone (160) according to claim 1, wherein the speaker duct (336) is coupled to at least another one of the plurality of ports (332); and/or wherein the microphone duct (338) is coupled to at least a different one of the plurality of ports (334).

3. The nose cone (160) according to claim 1 or 2, wherein the speaker duct (336) and/or the microphone duct (338) is at least partly incorporated into the cone body (165).

4. The nose cone (160) according to any one of the preceding claims, wherein the cone body (165) comprises essentially a truncated hollow cone shape.

5. The nose cone (160) according to any one the preceding claims, wherein the plurality of ports (230, 332, 334) are circularly arranged on a ring circle on the top surface (225) of the top portion (220).

6. The nose cone (160) according to one of claims 2 to 5, wherein the at least another one of the plurality of ports (332) and the at least a different one of the plurality of ports (334) can enclose a circular arc of more than 90°, preferably of more than 120° and more preferably of 180° from each other.

7. The nose cone (160) according to one of claims 2 to 6, wherein at least another one of the plurality of ports (332) and the at least a different one of the plurality of ports (334) are adjacent to each other.

8. The nose cone (160) according to one of the preceding claims, wherein the at least one light guide element (144) comprises a plurality of optical fibers.

9. The nose cone (160) according to claim 8, wherein the optical fibers are evenly and/or symmetrically distributed around the perimeter of the cone body (165).

10. The nose cone (160) according to one of the preceding claims, wherein the cone body (165) is at least partly made from an epoxy material.

11. The nose cone (160) according to one of the preceding claims, wherein the cone body (165) comprises an axial opening (340) configured to transmit light reflected from a target structure from the top portion (220) to the base portion (210).

12. A vibrometry measurement system comprising:
a nose cone (160) according to any of claims 1 to 11;
a speaker (152) sound transmitting connected to a speaker duct (336) of the nose cone (160), to provide an acoustic stimulus to a structure;
a microphone (154) connected to the microphone duct (338) of the nose cone (160) configured to receive an acoustic pressure from a structure;
at least one light guide element configured to illuminate a target structure;
an OCT subsystem configured to measure a response signal from vibrations in a structure of the patient, wherein the OCT subsystem comprises an OCT light element and an OCT sensor system.

13. The system according to claim 12, wherein the OCT light element from the OCT sensor system illuminates the structure through the axial opening of the nose cone (160).

14. A method for manufacturing a nose cone (160) for a vibrometry measurement system comprising the steps of:
providing a cone shaped mounting part (902) having a plurality of recesses (904) on its outer surface configured for mounting a light guide, a speaker tube and a microphone tube;
inserting the light guide, the speaker tube and the microphone tube into the recesses (904) of the mounting part (902);
securing the light guide, the speaker tube and the microphone tube in the recesses (904) by means of a molding step.

15. The method according to claim 14, wherein the method further comprises:
providing an auxiliary mounting portion providing radial support of the light guide, the speaker tube, and/or the microphone tube.

16. The method according to claim 15, wherein the light guide, the speaker tube and/or the microphone tube are fixed at the auxiliary mounting portion.
